Europäisches Patentamt

European Patent Office

Office européen des brevets

(11) Publication number: **0 489 442 A1**

## EUROPEAN PATENT APPLICATION

(21) Application number: **91120963.3**

(22) Date of filing: **06.12.91**

(51) Int. Cl.5: **C12Q 1/70**, C12Q 1/68, C07H 21/04, C12P 19/34

(30) Priority: **06.12.90 IT 2231290**

(43) Date of publication of application:
**10.06.92 Bulletin 92/24**

(84) Designated Contracting States:
**AT BE CH DE DK ES FR GB GR IT LI LU NL SE**

(71) Applicant: **SCLAVO S.p.A.**
**Via Fiorentina 1**
**I-53100 Siena(IT)**

(72) Inventor: **Mazzatenta, Carlo**
**Residing in Via A. Diaz, 60**
**I-65010 Colle Corvino, (Prov of Pescara)(IT)**
Inventor: **Ricci, Stefano**
**Residing in Via Massetana, 13**
**I-53100 Siena(IT)**
Inventor: **Ratti, Giulio**
**Residing in Vico Alto**
**I-53100 Siena(IT)**

(74) Representative: **Gervasi, Gemma et al**
**NOTARBARTOLO & GERVASI Srl Viale**
**Bianca Maria 33**
**I-20122 Milan(IT)**

(54) Synthetic oligonucleotides useful for the diagnosis of infections from different types of virus of the papilloma group and usage thereof.

(57) There are described synthetic oligonucleotides suitable both as primers for the amplification of DNA segments from different human Papillomaviruses and as probe for typing said amplified segments and their use for manufacturing suitable diagnostic kits.

## Technical field of the invention

The present invention relates to synthetic oligonucleotides suitable both as primers enabling the amplification of DNA segments from different human Papillomaviruses and as probes enabling the typing of the above cited amplified segments and to their use for the manufacturing of suitable diagnostic kits.

## The State of the art

Human Papillomavirus (HPV) is a group of viruses recognized since long time as the aetiological agent of benign cutaneous or mucosal pathologies such as warts and sharp condylomas. Moreover they can give rise also to infections and/or lesions which show a high likelihood to develop into tumours. According to the convention that a clinical isolate of HPV is regarded as novel when it exhibits, in a liquid phase hybridisation assay, less than 50% homology with regard to any known type, 60 different types HPV have been evidenced so far. In general the HPV may be differentiated in "cutaneous" and "mucosal", namely they may be distinguished in virus types which preferentially infect the cute and virus types which, on the contrary infect the mucosa. Probably the most important differentiation is however that discriminating the Papillomaviruses in "oncogenic" and "non-oncogenic". In fact a lot of clinical and laboratory experimentations have put in evidence that some HPV types may be involved in the genesis of epithelia tumours either cutaneous or, specifically, of the genital apparatus (male or female). Within the ambit of the HPV infecting more specifically the mucosae, the most frequent "non-oncogenic" HPV (associated to benign lesions such as sharp condylomas) are of the type 6 and 11 (low risk) whereas the "oncogenic" ones, namely those associated with pre-neoplastic lesions (CIN I, II and III) and neoplastic lesions, specifically in the portio uterina, are of the types 16 and 18 (high risk) and of the types 31, 33, 35 and 42 (intermediate risk). Because of the risks associated with these infections, it has been tried to develop more and more accurate and sensitive techniques allowing a reliable diagnosis of the presence and of the type of the viruses in the different tissues. At present the main techniques enabling the detection of the presence and the identification of the molecular type of the HPV are:

1) analysis of the restriction patterns by the known technique Southern Blot (S.B.);
2) hybridisation with suitable probes according to the known technique Dot-Blot (D.B.);
3) hybridisation in situ (I.S.);
4) hybridisation in situ on filter (FISH);
5) Polymerase Chain Reaction (PCR).

The first four techniques above involve however different drawbacks such as: the necessity of employing strongly radiolabelled probes and of availing of a significant amount of the DNA from the sample under assay (from 2 to 10 μg per test); the long time necessary for the analysis; incidental problems concerning the signal specificity; the necessity of availing of suitable biological material and the burden of the method which renders the technique itself more suitable for research purpose than for clinical routine (technique 3). The technique 5, which was recently discovered, is for sure the most promising technique for the development of a simple, rapid but first of all sensitive method for detecting and typing of HPV in cutaneous and mucosal lesions. This technique allows the diagnosis and the selective typing of each single known type; however, since the HPV types are more than 60, it would be desirable to avail of a test enabling the detection of the presence of the highest possible number of HPV or at least of those HPV which most frequently are at the basis of defined pathological states.

## Detailed description of the invention

The purpose of the present invention is to provide a solution to the aforementioned problems in that it allows the detection of more HPV with one single amplification reaction, thus permitting a decrease of the number of the necessary tests and needing reduced amounts of sample. The present invention therefore relates to synthetic oligonucleotides, the synthesis thereof and their application:

- as primer in PCR tests for amplifying in one single test the DNA from the highest possible number of HPV types infecting the genital mucosae, namely those able to correctly hybridise with the most frequent genital HPV types (HPV 6, 11, 16, 18, 31, 33 and 42);
- as a probe to recognize, among all the PCR positive types, which is the specific and unique infecting HPV type among type 6, 11, 16, 18, 31, and 33. By preparing the primers of the present invention it was considered that the sequences useful for the amplification had to be found not to much apart one from another in order not to impair the PCR but in any case sufficiently apart to allow the easy electrophoretic identification of the amplified DNA and to facilitate the discrimination of the different amplified virus types (typing). The typing may be performed:
- by means of the analysis with restriction enzyme (for instance DraI) capable or not of cleaving the amplified sequence in such a way that the analysis of the resulting fragments allows the univocal identification of the amplified sequence; or preferably:
- by means of specific oligonucleotide probes selected within the amplified sequences. Moreover the two primers disclosed in the present invention have been found to allow, in one single reaction, not only the amplification of the aforementioned genital types but also of some other types (HPV 1, 2, 3, and 4) which, in selected DNA segment, are sufficiently homologous with them . The DNA fragments produced by the PCR making use of the primers according to the invention are of about 1000 bp (1400 in the case of HPV 4).

Experimental section

Detection of conservative sequences along the DNA strand from different types of sequenced HPV.

At the beginning two domains along the genomic nucleotide sequences of HPV 6., 11, 16, 18, 31 and 33 (i.e. among those that more frequently are involved in the genesis of benign and potentially malign lesions in the genital apparatus) were identified (by means of the programs of the Wisconsin University Genetics Computer Group). Said domains exhibit a sufficient degree of homology and comprise conservative sequences of at least 12 contiguous bases. Moreover such domains are separated one from another by a distance suitable for PCR performance (i.e about 1000 bases). In Table I and II, reported hereinafter, there are respectively illustrated 50 bases fragments from HPV 6, 11, 16, 18, 31 and 33 employed to identify the domains from which the left and right primers may be derived. The highly conservative blocks (boldface) and the good homology of the framing sequences can be appreciated; in particular it is evident from Table II that the bases completely conserved in all the six considered HPV types are as many as 12; a good homology is moreover noted among the further 7 bases. In the cited Tables, the line designated as "CONSENSUS" represents the average of the six matched sequences, wherein the capital letters evidence completely conservative positions, the small letters evidence the nucleotide prevalent in that position and the point evidences the absence of any preferential nucleotide. Thus the CONSENSUS indicates the degree of homology in each position of the sequences. The underlined part highlights the sequence fragment which was subsequently employed to define the structure of the selected primers.

```
TABLE I

Hpv31sx      GAGTGACC  GAAAGTGGT-  GAACCGAAAA  CGGTTGGTAT  A

Hpv33sx     GGGTGTAACC  GAAAGCGGTT  CAACCGAAAA  CGGTGCATAT  ATAAAGCAAA

Hpv18sx     GGAGTAACCG  AAAACGGTCG  GGACCGAAAA  CGGTGTATAT  AAAAGATGTG

Hpv16sx     GGGCGTAACC  GAAATCGGTT  GAACCGAAAC  CGGTTAGTAT  AAAAGCAGAC

Hpv11sx     AGGAGGGACC  GAAAACGGTT  CAACCGAAAA  CGGTTATATA  TAAACCAGCC

Hpv6sx      AGGAGGGACC  GAAAACGGTT  CAACCGAAAA  CGGTTGTATA  TAAACCAGCC

CONSUNSUS   gGgagt.aCc  gAAA.cGgtt  .aACCGAAAa  CGGTt..tat  aaAA.cag.c
```

The position of the fragments in Table I along the complete genomic sequence is reported below (the origin for the coordinates of each types are those adopted by the International Data Bank GenEmb1):

```
HPV  6 sx    from nucleotide  28     to nucleotide  77

HPV 11 sx    from nucleotide  28     to nucleotide  77

HPV 16 sx    from nucleotide  28     to nucleotide  77

HPV 18 sx    from nucleotide  36     to nucleotide  85

HPV 31 sx    from nucleotide  33     to nucleotide  70

HPV 33 sx    from nucleotide  32     to nucleotide  81
```

TABLE II

```
Hpv31dx          AGGCT GTGCAGGTTC TAAAACGAAA GTATGTAGGT AGTCCTTTAA

Hpv33dx    TTTAAATGCT GTGTGTGCAC TAAAACGAAA GTTTGCCGCA TGTTCACAAA

Hpv18dx    TGCACAAGTG TTGCATGTTT TAAAACGAAA GTTTGCAGGA GGCAGCACAG

Hpv16dx    TAGAGATGCA GTACAGGTTC TAAAACGAAA GTATTT-GGT AGTCCACTTA

Hpv11dx    TTATGCGACT GTGCAGGACC TAAAACGAAA GTATTTAGGC AGTCCATATG

Hpv6dx     TTATGCGACT GTGCAGGACC TAAAACGAAA GTATTTAGGT AGTCCATATG

CONSENSUS  Ttaagaggct gTgcagGttc TAAAACGAAA GTaT.taGgt agtccataag
```

The position of the HPV fragments along the sequenced genome is reported below:

```
HPV  6 dx    from nucleotide  1056    to nucleotide  1105

HPV 11 dx    from nucleotide  1056    to nucleotide  1105

HPV 16 dx    from nucleotide  1101    to nucleotide  1150

HPV 18 dx    from nucleotide  1147    to nucleotide  1196

HPV 31 dx    from nucleotide  1100    to nucleotide  1144

HPV 33 dx    from nucleotide  1112    to nucleotide  1161
```

It should be noted that the sequences in the given alignments are in the 5'-> 3'direction and refer to the strand reported in the data bank and conventionally designated as superior strand, whereas the sequence of the primer synthesized for purpose of amplification will be a copy of the complementary strand (inferior) and therefore of opposite polarity as that reported in the Tables. Therefore the oligonucleotide to be synthesized will show the completely conservative part of the sequence at the 3' end, which is important for the PCR mechanism. The sequences selected to be used as primers for the amplification have been produced in such a way not to favour one specific HPV type but rather to deviate from each HPV type with two or three bases. In Table III and IV there are reported the alignments of the DNA bases, for each HPV, corresponding to the underlined sequences in Tables I and II, namely on the left (Table I) and on the right (Table II) and the thus designed correspondent primers which are indicated with the designations PRISX and PRIDX

4

TABLE III

```
Hpv31sx    46 - GGT-GAACCGAAAACGGT - 62

Hpv33sx    48 - GGTtcAACCGAAAACGGT - 65

Hpv18sx    52 - GtcGGgACCGAAAACGGT - 69

Hpv16sx    44 - GGTtGAACCGAAAcCGGT - 61

Hpv11sx    44 - GGTtcAACCGAAAACGGT - 61

Hpv6sx     44 - GGTtcAACCGAAAACGGT - 61

PRIMER LEFT    5` - GGTGGAACCGAAAACGGT - 3`(PRISX)
```

TABLE IV

```
Hpv31dx    1097 - ATTTTGCTTTCATACATCC - 1115

Hpv33dx    1132 - ATTTTGCTTTCAaAcGgCg - 1150

Hpv18dx    1167 - ATTTTGCTTTCAaAcGTCC - 1185

Hpv16dx    1121 - ATTTTGCTTTCATAAacCa - 1139

pv11dx    1076 - ATTTTGCTTTCATAAaTCC - 1094

Hpv6dx    1076 - ATTTTGCTTTCATAAaTCC - 1094

PRIMER RIGHT    3' - ATTTTGCTTTCATAAGTCC - 5'(PRIDX)
```

Also in this case the bases represented by small letters within the sequences show differences between the primer and the natural sequence. The numbers on the side of the sequences show the position wherein each HPV fragment may be identified along the published sequence, however keeping in mind that in Table IV the sequences belong to the strand complementary to those of Table II. In Table V there are reported the length of the fragments to amplify relative to the positions which will hybridize with the primers.

TABLE V

| HPV 6 | from nucleotide 44 to nucleotide 1094 | | | 1050 base pairs | |
|---|---|---|---|---|---|
| HPV 11 | " | 44 | " | 1094 | 1050 | " |
| HPV 16 | " | 44 | " | 1139 | 1095 | " |
| HPV 18 | " | 36 | " | 1185 | 1149 | " |
| HPV 31 | " | 46 | " | 1115 | 1069 | " |
| HPV 33 | " | 48 | " | 1150 | 1102 | " |

Selection of oligonucleotides suitable for the synthesis of type specific probes

The segments to be amplified were compared one to another in order to determine the sequences of the oligonucleotide probes able to specifically recognize the corresponding HPV type only. The selection was carried out in such a way that the probes, while maintaining their discrimination ability, could hybridize to the corresponding target at the same optimum temperature in order to semplify the practical execution of the assay. It is however evident that other sequences could be selected according to analogous criteria. The sequences selected for the experimental examples are illustrated in Table VI.

### TABLE VI

| | |
|---|---|
| PRB6 | 5'- AGGTAAAACATATACTAACCA - 3' |
| PRB11 | 5'- ACTAAAGCACATATTGGGAA - 3' |
| PRB16 | 5'- AAAGACATCTGGACAAAAAG - 3' |
| PRB18 | 5'- ATCCAGCAGAAAAACTTAGA - 3' |
| PRB31 | 5'- ATTGACAAACAAAGGTATATG - 3' |
| PRB33 | 5'- AACGACATGTGGATTTAAAC - 3' |

### Synthesis of the oligonucleotides

The oligonucleotides of the above disclosed sequence were synthesised using an automatic DNA synthesizer (Model 380 B Applied Biosystems Inc.) employing reagents and methods according to the producer's instructions. The same was for the reagent Aminolink-2 used to obtain the terminal biotinisation of the same oligonucleotides to be used as probes.

### Experiences on a panel of cloned HPV

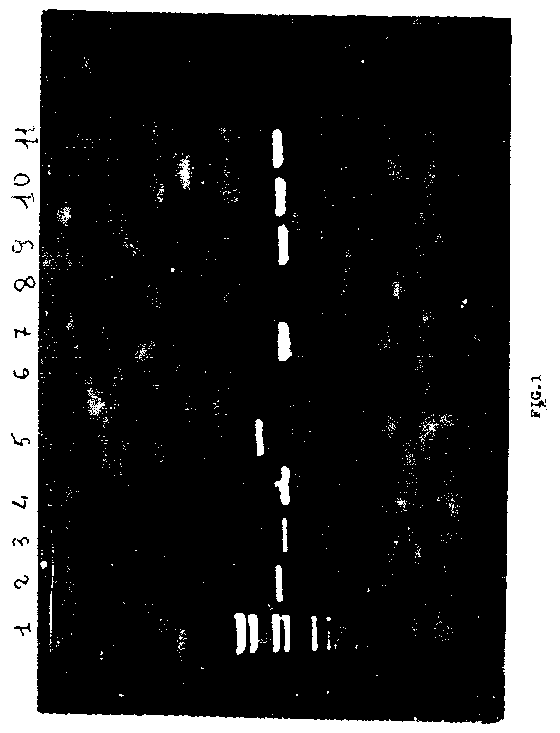

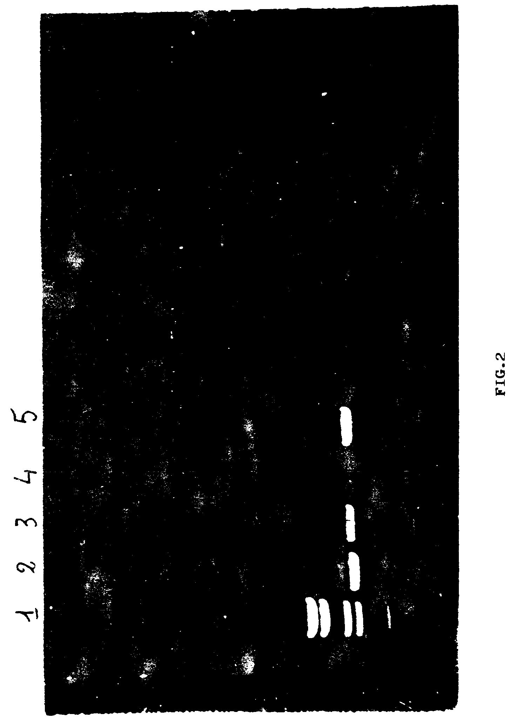

The control of the PRISX and PRIDX primer feasibility was achieved by carrying out amplification tests employing as target DNA the cloned genomes of some HPV. The materials used for the amplification, i.e. reaction buffer, nucleotides and the enzyme Taq polymerase were those supplied by the firm Perkin Elmer - Cetus for the kit GenAmp. The reaction mixtures consisted of 25 mM TRISHCl, pH 9.3, 50 mM KCl, 2 mM $MgCl_2$, 1 mM DTT, 200 $\mu$M of each dATP, dTTP, dCTP, dGTP 50 pM of each PRISX and PRIDX, 0.5 U of Taq-polymerase and 1 ng of plasmid comprising the cloned HPV as target DNA. The amplification conditions were the following: 7 amplification cycles at 94°C for 40", 37°C for 40" 72°C for 2 minutes, followed by 30 cycles at 94°C for 40", 50°C for 40", 72°C for 2 minutes. Thereafter, 10 $\mu$l amplification mixture were analyzed on agarose gel 1% in TAE to verify the achievement of the DNA amplification and the size of the obtained bands. By applying said specific amplification conditions, the amplification products of the HPV type 1, 2, 3, 4, 6, 11, 16, 18, 31, 33, and 42 could be obtained. On the contrary the HPV type 5, 8 and 35 did not result in amplified products. Figure 1 illustrates the analysis on agarose gel of the obtained products wherein lanes 2 to 11 correspond to the HPV 1, 2, 3, 4, 5, 6, 8, 11, 16 and 18, while on lane 1 the molecular weight markers are reported (Boehringer Molecular Weight Markers VI). Figure 2, like Figure 1 illustrates the amplification of HPV 31, 33, 35 and 42 (corresponding to lanes 2 to 5). The relationship of the obtained bands with the corresponding HPV types was proved for HPV 6, 11, 16, 18, and 33 both by means of the restriction enzymes analysis and hybridisation tests with the above described type specific probes.

### Typing of amplified HPV

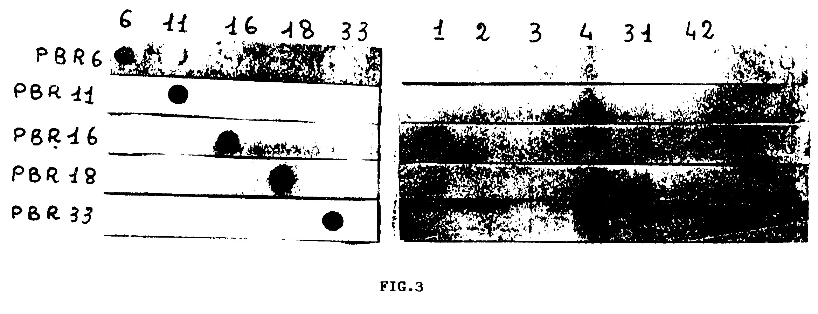

The aforementioned amplified segments were typed by means of the above disclosed oligonucleotides. Such oligonucleotides were suitably prepared by employing during the synthesis the reagent Aminolink-2 (Applied Biosystems Inc.); they were thus labelled by terminal biotinisation. For purpose of typing, 5 $\mu$l of amplification reaction were collected, adjusted to a volume of 100 $\mu$l with water, and the DNA denatured for 5 minutes at room temperature upon addition of 75 $\mu$l of 0.5 M NaOH, 2.5 M NaCl solution. Then the solution was neutralized by adding 25 $\mu$l of 1 M TRIS solution, pH 7.5. Such a procedure was carried out fivefold for each amplified HPV type. Thereafter the denatured DNA was blotted on a Nylon membrane using a Dot-Blot equipment. After blotting the membrane was submitted to U.V. for 5' to cause the DNA anchor to the Nylon. Then each series of amplified sample was hybridized with a different biotinilated probe specific for a given HPV type. The hybridisation was carried out for one hour at 50°C in 3 ml of hybridisation mixture having the following composition: 6xSSC (1xSSC = 0.15 M NaCl, 0.015 M sodium citrate), 0.5% SDS, 5 x Denhard solution, 50 pM/ml of biotinilated probe. After hybridisation, the membranes was washed for 10 min in 2xSSC at 50°C to remove the not hybridized oligonucleotides. The presence of biotin was then detected by means of the complex streptavidin / biotinilated alkaline phosphatase as present in commercial kits, for example Gibco-BRL. Figure 3 shows the results of a hybridisation experience on the amplified DNA segments obtained from genomic DNA from HPV cloned in bacterial vectors. In Figure 3, there are represented strips of the Nylon membrane submitted to hybridisation with 5 of the aforementioned probes (i.e. PRB 6, 11, 16, 18 and 33). Samples of amplified DNA from HPV 6, 11, 16, 18, 33, 1, 2, 3, 4, 31 and 42 (from the left to the right in the figure) were loaded onto each strip.

Detection and typing of HPV in clinical samples

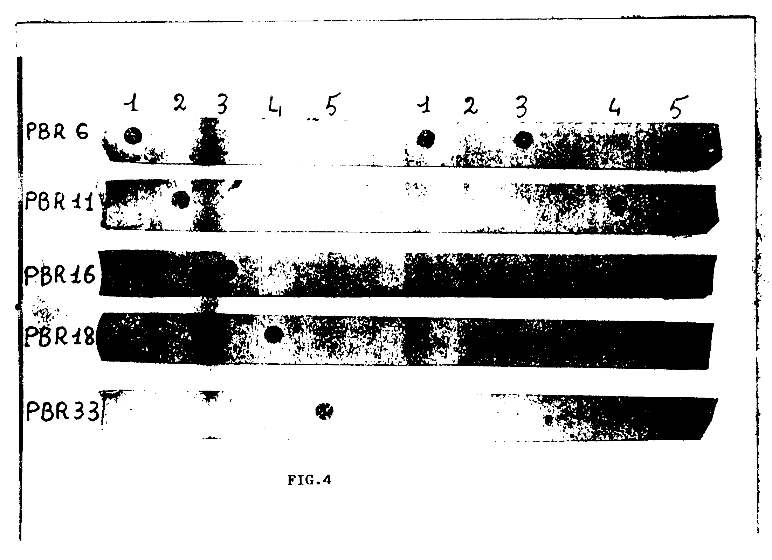

The above described procedure was employed for HPV detection and typing in cytological specimens from patients with suspected HPV infections. The material collected by scraping the mucosa under test, was dispersed in 0.5 ml of lysis solution comprising 10 mM TRIS-HCl, 150 mM NaCl, 10 mM EDTA, 0.6% SDS, 100 $\mu$l/ml K proteinase. The samples were incubated at 37°C for 3-5 hours, then kept in frozen state at minus 20°C until used. The DNA was extracted from said samples with phenol-chloroform and by ethanol precipitation. After precipitation, the pellet was resuspended in 100 $\mu$l of water and 25 $\mu$l of such a solution were used as target DNA for the PCR run by the above cited conditions. After reaction, 10 $\mu$l of mixture were analysed on agarose gel obtaining results similar to those illustrated in Figure 1 and 2. The samples resulting positive were typed by the above disclosed Dot-Blot technique with biotinilated probes. Figure 4 illustrates by way of example the results obtained from 5 clinical samples. On the left side there are reported the five controls (like as in figure 3) whereas on the right side there are reported the results obtained from the five clinical samples.

**Claims**

1. Method for the detection and typing of Papilloma virus in biological samples wherein a segment of the Papilloma virus DNA, if present, is amplified by PCR using the two primers having the following nucleotide sequence :
5' - GGTGGAACCGAAAACGGT - 3' and 3' - ATTTTGCTTTCATAAGTCC - 5'
and afterwards is indentified with hybridisation techniques using probes having the following nucleotide sequences:

5'-AGGTAAAACATATACTAACCA-3', 5'-ACTAAAGCACATATTGGGAA-3',

5'-AAAGACATCTGGACAAAAAG-3', 5'-ATCCAGCAGAAAAACTTAGA-3',

5'-ATTGACAAACAAAGGTATATG-3' and 5'-AACGACATGTGGATTTAAAC-3'

suitably modified for recognizing complementary homologous DNAs. .

2. Method according to claim 1, wherein said segment to be amplified is represented by the portion of HPV-6 DNA comprised between nucleotide 44 and nucleotide 1094.

3. Method according to claim 1, wherein said segment to be amplified is represented by the portion of HPV-11 DNA comprised between nucleotide 44 and nucleotide 1094.

4. Method according to claim 1, wherein said segment to be amplified is represented by the portion of HPV-16 DNA comprised between nucleotide 44 and nucleotide 1139.

5. Method according to claim 1, wherein said segment to be amplified is represented by the portion of HPV-18 DNA comprised between nucleotide 36 and nucleotide 1185.

**6.** Method according to claim 1, wherein said segment to be amplified is represented by the portion of HPV-31 DNA comprised between nucleotide 46 and nucleotide 1115.

**7.** Method according to claim 1, wherein said segment to be amplified is represented by the portion of HPV-33 DNA comprised between nucleotide 48 nucleotide 1150.

**8.** Method according to claim 1, wherein said probes are modified through a terminal biotinisation.

**9.** Method according to claim 8, wherein the terminal biotinisation is carried out by the technique known as Dot-Blot hybridisation.

**10.** Kit for the application of the method according to claim 1, containing said primers, said probes and the other suitable reagents for use in the PCR and hybridisation techniques.

FIG.1

FIG.2

FIG.3

FIG. 4

European Patent Office

Application Number

EP 91 12 0963

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 301 968 (IRE-CELLTARG S.A.) <br> * Abstract; page 8, line 57 - page 11 * <br> --- | 1-10 | C 12 Q 1/70 <br> C 12 Q 1/68 <br> C 07 H 21/04 <br> C 12 P 19/34 |
| A | WO-A-9 002 821 (CETUS CORP.) <br> * Pages 15-22 * <br> --- | 1-10 | |
| A | WO-A-8 905 357 (MICROPROBE CORP.) <br> * Abstract; page 21 * <br> --- | 1 | |
| P,A | EP-A-0 425 995 (ABBOTT LABORATORIES) <br> * Abstract; claims 1-5 * <br> --- | 1-10 | |
| A | WO-A-8 909 940 (ONCOR INC.) <br> * Whole document * <br> --- | 1 | |
| P,A | EP-A-0 402 132 (TAKARA SHUZO CO., LTD) <br> * Abstract; page 2 * <br> ----- | 1-10 | |

TECHNICAL FIELDS SEARCHED (Int. Cl.5)

C 12 Q

The present search report has been drawn up for all claims

| Place of search | Date of completion of the search | Examiner |
|---|---|---|
| THE HAGUE | 13-02-1992 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................

& : member of the same patent family, corresponding document

EPO FORM 1503 03.82 (P0401)